Europäisches Patentamt

European Patent Office

Office européen des brevets

(19)

(11) **EP 1 175 472 B1**

(12) **EUROPÄISCHE PATENTSCHRIFT**

(45) Veröffentlichungstag und Bekanntmachung des Hinweises auf die Patenterteilung:
**28.01.2004 Patentblatt 2004/05**

(21) Anmeldenummer: **00910872.1**

(22) Anmeldetag: **22.03.2000**

(51) Int Cl.$^7$: **C10J 3/00**, C07C 29/151, C10J 3/16

(86) Internationale Anmeldenummer:
**PCT/EP2000/002517**

(87) Internationale Veröffentlichungsnummer:
**WO 2000/058421 (05.10.2000 Gazette 2000/40)**

(54) **VERFAHREN UND VORRICHTUNG ZUR ERZEUGUNG VON ENERGIE BZW. METHANOL**

METHOD AND DEVICE FOR PRODUCING ENERGY OR METHANOL

PROCEDE ET DISPOSITIF PERMETTANT DE PRODUIRE DE L'ENERGIE OU DU METHANOL

(84) Benannte Vertragsstaaten:
**AT BE CH CY DE DK ES FI FR GB GR IE IT LI LU MC NL PT SE**

(30) Priorität: **26.03.1999 DE 19913786**

(43) Veröffentlichungstag der Anmeldung:
**30.01.2002 Patentblatt 2002/05**

(73) Patentinhaber:
• **Prestel, Michael
71065 Sindelfingen (DE)**
• **Krebs, Thomas
71067 Sindelfingen (DE)**

(72) Erfinder: **GERSTWEILER, Herbert
D-71067 Sindelfingen (DE)**

(74) Vertreter: **Hössle, Markus, Dipl.-Phys.
Hössle Kudlek & Partner
Postfach 10 23 38
70019 Stuttgart (DE)**

(56) Entgegenhaltungen:
**EP-A- 0 257 018          EP-A- 0 699 651
DE-A- 3 228 532          DE-A- 19 734 259
US-A- 3 763 205          US-A- 4 087 449
US-A- 4 372 755          US-A- 4 526 903**

• **Pollesel, P. "Energy Recovery from Waste: the Application of Gasification Technologies", La Chimica e L'Industria, 1996, no. 5, pages 603-607**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 175 472 B1

**Beschreibung**

Die vorliegende Erfindung betrifft ein Verfahren und eine Vorrichtung zur Erzeugung von Energie sowie ein Verfahren und eine Vorrichtung zur Erzeugung von Methanol.

Es sind bereits zahlreiche Verfahren und Vorrichtungen zur Erzeugung von Energie und/oder Methanol aus organischen Rohstoffen bekannt.

Einige dieser Verfahren und Vorrichtungen beruhen auf dem Prinzip, daß ein organisches Material einer kontrollierten Oxidation unterzogen wird, wobei der Energieträger Wasserstoff entsteht. Der gewonnene Wasserstoff wird entsprechend dem jeweiligen Strombedarf einer Brennstoffzelle zugeführt und verstromt. Da eine Speicherung von nicht benötigtem gasförmigem Wasserstoff aufwendig ist und insbesondere bei einem Einsatz in Privathaushalten hohen Sicherheitsanforderungen genügen muß, wird Wasserstoff zur Speicherung seines Energiegehalts üblicherweise mit Kohlenmonoxid oder Kohlendioxid katalytisch zu dem flüssigen Energieträger Methanol umgesetzt, aus dem bedarfsgerecht durch eine Reformierreaktion Wasserstoff zurückgewonnen und verstromt werden kann.

In der DE 196 44 684 A1 ist ein Verfahren zur Speicherung von in Form von Wasserstoff vorliegender Energie offenbart, wobei insbesondere aus Abgasen anfallendes Kohlendioxid nach Mischung mit dem Wasserstoff in einem Reaktor zu den Energieträgern Methan, Methanol oder Ethanol umgesetzt wird.

In der DE 44 30 750 C2 sind ein Verfahren und eine Vorrichtung zur Erzeugung elektrischer Energie aus Biorohstoffen offenbart, wobei aus den Biorohstoffen durch Teiloxidation mit einem sauerstoffhaltigen Vergasungsmittel in einem Oxidationsreaktor ein wasserstoff- und kohlenmonoxidhaltiges Brennstoffrohgas erzeugt wird. Aus dem Brennstoffrohgas wird der Wasserstoffanteil durch Reaktion mit einem Speichermaterial, insbesondere mit einem sogenannten Hydridspeicher oder mit Metalloxiden, zwischengespeichert, so daß eine schnelle, bedarfsgerechte Entnahme des Wasserstoffs aus dem Speicher und die Weiterleitung zur Verstromung in ein Brennstoffzellenmodul möglich ist.

In der DE 197 34 259 A1 sind ein Verfahren und eine Vorrichtung zur Erzeugung von Wasserstoff im Zuge der Vergasung von Biorohstoffen offenbart, wobei der Biorohstoff in Anwesenheit von zugeführtem Wasserdampf in einem Verdampfungsreaktor zu einem Wasserstoff enthaltenden Rohgas vergast wird. Der Wasserstöffanteil des Rohgases wird in besonders hoher Reinheit von dem Restgas abgetrennt und kann entweder in einer Brennstoffzelle verstromt, durch Synthese mit Kohlendioxid als Methanol gespeichert oder für andere Zwecke verwendet werden. In diesem Verfahren wird zur Erhöhung des Wirkungsgrades auch der Energiegehalt des wasserstoffarmen Restgases genutzt, indem es entweder in einem geschlossenen Kreislauf zur Erzeugung von Wärmeenergie für die Wasserdampferzeugung oder außerhalb des Kreislaufs verbrannt wird.

Aus der EP 0 257 018 A2 ist ein Verfahren zur thermischen Verwertung von Abfällen und/oder Abfallbrennstoffen bekannt, bei dem die Abfälle und/oder Abfallbrennstoffe einem mit Gas und/oder Öl direkt beheizten Vergasungsreaktor zugeführt werden und die aus dem Vergasungsreaktor abgezogenen Gase einer Brennkammer zur Energieerzeugung, insbesondere zur Dampferzeugung, zugeführt werden. Ein Teil der Rauchgase aus der Brennkammer wird unter Druck dem Vergasungsreaktor zurückgeführt.

Aus der DE 32 28 532 A1 ist ein Verfahren zur Verschwelung und Vergasung von kohlenstoffhaltigen Feststoffen bekannt, wobei zur Beheizung der Reaktoren u.a. Heißgase verwendet werden, die in einer Brennkammer durch Verbrennung eines Teils des in der Schwelzone erzeugten Schwelgases erzeugt werden.

In "Energy Recovery From Waste: The Application of Gasification Technologies", La Chimica e l'Industria, 1996, Nr. 5, Seiten 603-607 gibt P. Pollesel einen Überblick über die Anwendung von Vergasungsverfahren zur Gewinnung von Energie aus Abfällen, insbesondere aus Biomasse. Pollesel erwähnt u.a., daß die bei der Vergasung erzeugten Gase in einer Gasturbine oder in einem Dieselmotor verbrannt werden können, um mechanische Energie zu erzeugen, die nachfolgend in elektrische Energie umgewandelt werden kann.

Demgegenüber liegt der vorliegenden Erfindung die Aufgabe zugrunde, ein Verfahren und eine Vorrichtung zur Erzeugung von Energie bzw. Methanol zu schaffen, bei dem bzw. der zur Erzielung eines besonders wirtschaftlichen Betriebs eine optimierte Ausnutzung des Energiegehalts des organischen Ausgangsmaterials erfolgt und gleichzeitig die Anteile freigesetzter bzw. gespeicherter Energie bedarfsgerecht regelbar sind.

Zur Lösung dieser Aufgabe werden ein Verfahren nach Anspruch 1 und eine Vorrichtung nach Anspruch 8 vorgeschlagen.

Das erfindungsgemäße Verfahren zur Erzeugung von Energie umfaßt die folgenden Schritte:

1. Verbrennen von Brennstoff in einer Brennkraftmaschine unter Erzeugung von mechanischer Energie und heißem kohlendioxid- und wasserdampfhaltigem Abgas,

2. Reduktion der heißen Abgaskomponenten Kohlendioxid und Wasser in einem zugeführtes organisches Material enthaltenden thermo-chemischen Reaktor zu einem Synthesegas, das Kohlenmonoxid und Wasserstoff enthält, wobei das organische Material oxidiert wird,

3. Synthetisieren von Methanol aus dem Synthesegas und Abscheiden des Methanols

4. Zuführen des in Schritt 3 nicht umgesetzten Synthesegases und/oder des synthetisierten Methanols in die Brennkraftmaschine (12).

Die Schritte des erfindungsgemäßen Verfahrens sind für einen Kreislaufbetrieb ausgelegt, aus dem an verschiedenen Stellen Energie in Form von elektrischer Energie (Strom), Wärmeenergie und gespeicherter chemischer Energie (Methanol) entnommen und/oder zugeführt werden kann, wobei die Entnahme und die Zufuhr bedarfsgerecht regelbar sind und somit eine optimierte Nutzung des Energiegehalts des organischen Ausgangsmaterials erzielbar ist.

Erfindungsgemäß wird somit ein Brennstoff, der vorzugsweise kohlenwasserstoffhaltig und flüssig bzw. gasförmig ist, bei Zufuhr von Luft bzw. Sauerstoff in einer Brennkraftmaschine unter Erzeugung von mechanischer Energie, Abgas und Wärmeenergie verbrannt. Das aus der Brennkraftmaschine freiwerdende Abgas, das Kohlendioxid und Wasserdampf enthält, wird einem thermo-chemischen Reaktor zugeführt, in dem organisches Material, wie beispielsweise Biomasse, Kohle, organische Abfälle und dergleichen, vorhanden ist. In dem thermo-chemischen Reaktor wird Kohlendioxid und Wasser aus dem Abgasstrom aus der Brennkraftmaschine zu Kohlenmonoxid und Wasserstoff reduziert, während das organische Material oxidiert wird. Die für die Reaktion erforderliche Temperatur wird durch mitgeführte Wärmeenergie des Abgasstroms erzeugt. Sofern die durch das einströmende Abgas erzielbare Temperatur nicht ausreicht, um die Reaktion einzuleiten bzw. aufrechtzuerhalten, kann ein Teil des heißen Abgasstroms aus der Brennkraftmaschine von dem Gesamtstrom abgetrennt und als äußere Wärmezufuhr zur Beheizung des thermo-chemischen Reaktors vorgesehen werden und/oder durch Zufuhr von Sauerstoff bzw. Luft das für den Ablauf der Reaktion erforderliche Temperaturniveau erhöht werden. Vorteilhafterweise beträgt die Temperatur des heißen Abgases zwischen etwa 900 °C und etwa 1.000 °C. Somit wird das heiße Abgas aus der Brennkraftmaschine als wertvoller Energieträger direkt genutzt, während die durch die Brennkraftmaschine erzeugte mechanische Energie für weitere Zwecke zur Verfügung steht.

In dem thermo-chemischen Reaktor wird unter den geeigneten Druck- und Temperaturverhältnissen ein kohlenmonoxid- und wasserstoffhaltiges Synthesegas erzeugt. Das Synthesegas kann weitere nicht störende Bestandteile, wie beispielsweise Kohlendioxid oder Methan, und nicht erwünschte bzw. störende feste, flüssige und/oder gasförmige Bestandteile, wie beispielsweise Schwebstoffe oder schwefelhaltige Gase, enthalten. Die störenden bzw. nicht erwünschten Bestandteile können sich nachteilig auf weitere Schritte des erfindungsgemäßen Verfahrens auswirken, z.B. können sie eine Katalysatorvergiftung bewirken. Daher ist ein nachgeschalteter Filtrationsschritt zur Entfernung der nicht erwünschten bzw. störenden Bestandteile vorgesehen.

Das Kohlenmonoxid und Wasserstoff enthaltende, durch den Filtrationsschritt gereinigte Synthesegas aus dem thermo-chemischen Reaktor wird in einer Gasmischeinheit mit Luftsauerstoff oder Sauerstoff aus einem Sauerstofftank gemischt und der Brennkraftmaschine als Brennstoff zugeführt. Somit wird die Versorgung des thermo-chemischen Redoxprozesses mit heißem Abgas sichergestellt.

Sofern mehr Synthesegas hergestellt wird, als für den Betrieb der Brennkraftmaschine erforderlich ist, kann alternativ dazu aus dem Kohlenmonoxid und Wasserstoff enthaltenden, durch den Filtrationsprozeß gereinigten Synthesegas in einem Reaktor, der einen geeigneten Katalysator enthält, nach einem üblichen Verfahren aus Kohlenmonoxid und Wasserstoff bzw. Kohlendioxid und Wasserstoff in einer exothermen Reaktion der Energieträger Methanol synthetisiert werden. Neben Wärmeenergie entstehen als Reaktionsprodukte Methanol und gegebenenfalls Wasser sowie nicht umgesetztes Synthesegas. Die Reaktionsprodukte werden in einer nachgeordneten Kühleinheit abgekühlt. Das sich abscheidende Methanol kann in einen Tank geleitet und dort gespeichert werden. Nach Bedarf kann das gespeicherte Methanol als Brennstoff der Brennkraftmaschine zugeführt werden oder für weitere Zwecke, z.B. als Treibstoff für Kraftfahrzeuge zur Verfügung stehen.

Überschüssiges, nicht umgesetztes Syntheserestgas aus der Methanolsynthese wird wie oben beschrieben der Brennkraftmaschine zugeführt.

In Ausgestaltung der Erfindung wird die durch die Brennkraftmaschine erzeugte mechanische Energie mittels eines Generators in Strom umgewandelt. Der Strom kann entweder für die Versorgung eines Privathaushaltes zur Verfügung stehen, in einer Batterie gespeichert oder in das Stromnetz eingespeist werden.

In weiterer Ausgestaltung der Erfindung wird zur Stromerzeugung eine Brennstoffzelle in einer Brennstoffzelleneinheit über einen Gasmischer mit Sauerstoff und Wasserstoff versorgt, wobei der Sauerstoff aus einem Sauerstofftank zugeführt werden kann oder Luftsauerstoff sein kann und der Wasserstoff entweder aus dem durch den Filtrationsprozeß gereinigten Synthesegas stammt oder durch Reformierung aus dem gespeicherten Methanol gewonnen wird. Mit dieser Art der Stromerzeugung ist ein besonders hoher Wirkungsgrad für die Umwandlung von gespeicherter chemischer Energie in elektrische Energie erzielbar. Zudem besteht durch die Nutzung der chemischen Energie des gespeicherten Methanols die Möglichkeit zur Anpassung der Stromerzeugung an eine bestehende Last.

In besonders vorteilhafter Ausgestaltung der Erfindung kann in den einzelnen Verfahrensschritten entstehende Wärme, insbesondere die Abwärme der Brennkraftmaschine, des Generators, des Methanolreaktors und der Kühleinheit, einem Wärmespeicher zugeführt werden und beispielsweise für die Beheizung des thermo-chemischen Reaktors oder für die Beheizung von Räumen zur Verfügung stehen.

In weiterer besonders vorteilhafter Ausgestaltung der Erfindung sind die Stoff- und Energieströme über eine Steuerung, z.B. eine Mikroprozessor-Steuerung regelbar. Dies betrifft insbesondere die Regelung der Abgaszufuhr aus der Brennkraftmaschine in den thermo-chemischen Reaktor, die Regelung der Gasströme in der Gasmischeinheit, die Überwachung und Regelung der Parameter der Methanolsynthese, die Temperatur-Regelung bei verschiedenen Verfahrensschritten, die Regelung der Energieströme des Wärmespeichers und die Überwachung des Füllstands bzw. die Regelung der Zufuhr des organischen Materials in den thermo-chemischen Reaktor. Somit wird jederzeit eine optimale Versorgung der Elemente der einzelnen Verfahrensschritte mit Substraten und Energie erreicht. Gleichzeitig wird der Energiegehalt der Brennstoffe optimal genutzt und eine Anpassung von Energiegewinnung und Energiespeicherung an den jeweiligen Bedarf ermöglicht.

Zur weiteren Lösung der der Erfindung zugrundeliegenden Aufgabe wird eine Vorrichtung zur Erzeugung von Energie und/oder Methanol insbesondere zur Durchführung des vorstehend beschriebenen Verfahrens vorgeschlagen. Die erfindungsgemäße Vorrichtung umfaßt eine Brennkraftmaschine, einen der Brennkraftmaschine nachgeordneten thermo-chemischen Reaktor zur Reduktion von in der Brennkraftmaschine erzeugtem kohlendioxid- und wasserdampfhaltigem Abgas zu einem Synthesegas, das Kohlenmonoxid und Wasserstoff enthält. Zur Erzeugung von Energie ist eine Zuleitung des Synthesegases in die Brennkraftmaschine vorgesehen. Für die Erzeugung von Methanol ist ein dem thermo-chemischen Reaktor nachgeordneter Methanolreaktor zur Synthese von Methanol aus dem Synthesegas des thermo-chemischen Reaktors vorgesehen, wobei eine Zuleitung von Syntheserestgas und/oder Methanol aus der Methanolsynthese in die Brennkraftmaschine erfolgt.

Für die Erzeugung von Strom ist in vorteilhafter Ausgestaltung der Erfindung ein durch die Brennkraftmaschine antreibbarer Generator vorgesehen. Weiterhin kann eine dem thermo-chemischen Reaktor nachgeordnete Brennstoffzelleneinrichtung zur Erzeugung von Strom aus Methanol und/oder aus zumindest einem Teil des in dem thermo-chemischen Reaktor erzeugten Wasserstoffs vorgesehen sein.

Zur Speicherung von Energie ist in weiterer Ausgestaltung der erfindungsgemäßen Vorrichtung ein Wärmespeicher zur Speicherung von mit der Vorrichtung erzeugter Wärme vorgesehen. Der Wärmespeicher dient zur Speicherung insbesondere der Abwärme aus den exothermen Verfahrensschritten, beispielsweise der Brennkraftmaschine, des Generators, der Methanolsynthese und/oder der Kühleinheit. Die gespeicherte Abwärme wird in dem endothermen Verfahrensschritt im thermo-chemischen Reaktor oder für andere Zwecke genutzt. Weiterhin ist ein Methanoltank zur Speicherung des synthetisierten Methanols vorgesehen, wobei bei Bedarf das gespeicherte Methanol in die Brennkraftmaschine und/oder die Brennstoffzelleneinrichtung zuführbar ist.

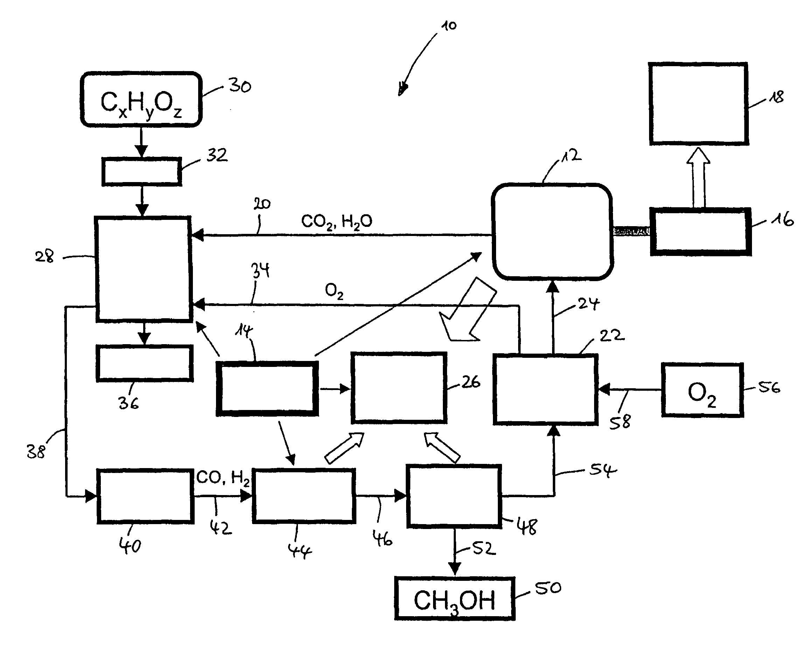

Weitere Vorteile und Ausgestaltungen der Erfindung ergeben sich aus der Beschreibung und der beiliegenden Zeichnung.

Die Erfindung ist anhand eines Ausführungsbeispieles in der Zeichnung schematisch dargestellt und wird im folgenden unter Bezugnahme auf die Zeichnung ausführlich beschrieben.

Die einzige Figur zeigt in stark schematischer BlockbildDarstellung eine erfindungsgemäße Vorrichtung zur Erzeugung von Energie und Methanol.

Die Figur zeigt eine erfindungsgemäße Vorrichtung 10 zur Erzeugung von Energie und Methanol mit einer Brennkraftmaschine 12, die einen kohlenwasserstoffhaltigen Brennstoff unter Erzeugung von mechanischer Energie, heißem Abgas und Abwärme verbrennt. Die erzeugte mechanische Energie wird mittels eines Generators 16 in Strom umgewandelt, welcher entweder in einer Batterie 18 gespeichert oder in das Stromnetz eingespeist oder direkt, beispielsweise in einem Privathaushalt, genutzt werden kann. Die Abwärme der Brennkraftmaschine 12 und die Abwärme des Generators 16 werden einem Wärmespeicher 26 zugeführt. Die Brennkraftmaschine 12 wird über eine Brenngasleitung 24 aus einem Gasmischer 22 mit dem gasförmigen Brennstoff und Sauerstoff versorgt.

Das aus der Brennkraftmaschine freiwerdende Abgas enthält Kohlendioxid und Wasser und sollte vorteilhafterweise eine Temperatur von etwa 900°C bis etwa 1.000°C aufweisen. Das heiße Abgas wird über eine Abgasleitung 20 in einen thermo-chemischen Reaktor 28 eingeleitet. In den thermo-chemischen Reaktor 28 wird über eine Schleuse 32 organisches Material aus einem Vorratsbehälter 30 eingefüllt. Der Füllstand des Behälters 30 sowie die durch die Schleuse 32 einzufüllende Menge des organischen Materials wird vorzugsweise durch eine Steuerung 14 geregelt.

Das in den thermo-chemischen Reaktor 28 eingeschleuste organische Material wird durch den heißen Abgasstrom aus der Brennkraftmaschine 12 auf etwa 900 °C bis etwa 1.000 °C erhitzt. Sofern die durch den Abgasstrom mitgeführte Wärmemenge nicht zur Erreichung der erforderlichen Temperatur ausreicht, wird ein Teil des heißen Abgasstroms abgetrennt und zur Beheizung des thermo-chemischen Reaktors 28 verwendet. Zusätzlich kann gespeicherte Wärmeenergie aus dem Wärmespeicher 26 eingesetzt werden. Weiterhin besteht die Möglichkeit, die Temperatur durch Zufuhr von Sauerstoff bzw. Luft aus dem Gasmischer 22 über eine erste Sauerstoffleitung 34 in den thermo-chemischen Reaktor 28 zu erhöhen. In dem thermo-chemischen Reaktor 28 wird bei den genannten Temperaturverhältnissen Kohlendioxid und Wasser zu einem Synthesegas reduziert, während das organische Material oxidiert wird.

Das Synthesegas wird über eine Synthesegasleitung 38 aus dem thermo-chemischen Reaktor abgeführt. Unter den in dem thermo-chemischen Reaktor 28 einzustellenden Temperaturund Druckverhältnissen enthält das Synthesegas hauptsächlich Wasserstoff und Kohlenmonoxid. Als Nebenbestandteile des ausgeschleusten Synthesegases können Kohlendioxid, Methan, Wasser und Schwebstoffe auftreten. Als weiteres Produkt der in dem thermo-chemischen Reaktor 28 ablaufenden Redoxreaktion fällt Asche an, die hauptsächlich aus mineralischen und nicht vollständig oxidierten Bestandteilen des ursprünglichen organischen Materials besteht. Die Asche fällt in einen Aschenbehälter 36. Der Füllstand des Aschenbehälters 36 und seine Leerung werden vorzugsweise durch die Steuerung 14 überwacht und geregelt.

Das in dem thermo-chemischen Reaktor 28 erzeugte Synthesegas wird über die Synthesegasleitung 38 über eine Filtrationseinheit 40 geleitet. In der Filtrationseinheit 40 wird das Synthesegas zunächst von festen und flüssigen Schwebstoffen befreit und vorzugsweise auch von weiteren z.B. schwefelhaltigen Minorkomponenten, die nachfolgende katalytische Schritte stören würden, befreit. Das über die Filtrationseinheit 40 gereinigte Synthesereingas, das hauptsächlich Wasserstoff und Kohlenmonoxid enthält, kann entweder direkt in die Gasmischeinheit 22 geleitet und von dort aus über die Brenngasleitung 24 der Brennkraftmaschine 12 als Brennstoff zugeführt werden oder einem Methanolreaktor 44 zugeführt werden. In dem Methanolreaktor 44 ist ein für die Methanolsynthese geeigneter Katalysator vorgesehen. Je nach Zusammensetzung des Synthesereingases können folgende Reaktionen ablaufen:

$$CO + 2H_2 \rightarrow CH_3OH$$

$$CO_2 + 3H_2 \rightarrow CH_3OH + H_2O$$

Als Produkte der Methanolsynthese werden Methanol und nicht umgesetzte Bestandteile des Synthesereingases über eine erste Reingasleitung 46 in einen Kühler 48 geleitet. In dem Kühler 48 scheidet sich Methanol und gegebenenfalls Wasser ab, während das verbleibende Gas über eine zweite Reingasleitung 54 in den Gasmischer 22 geführt wird, dort mit Sauerstoff bzw. Luft gemischt wird und über die Brenngasleitung 24 als Brennstoff der Brennkraftmaschine 12 zugeführt werden kann. Das sich in dem Kühler 48 abscheidende Methanol kann über eine Methanolleitung 52 in einen Methanoltank 50 überführt und dort gespeichert werden. Das gespeicherte Methanol steht bei Bedarf als Brennstoff für die Brennkraftmaschine 12 zur Verfügung oder kann für andere Zwecke, z.B. als Treibstoff für Kraftfahrzeuge zur Verfügung stehen. Die Abwärme aus dem Methanolreaktor 44 und dem Kühler 48 wird vorteilhafterweise in den Wärmespeicher 26 überführt und dort gespeichert.

Über die Steuerungseinheit 14 kann vorteilhafterweise auch die Gaszufuhr und Mischung in dem Gasmischer 22 geregelt werden. Dabei bezieht der Gasmischer 22 über eine zweite Sauerstoffleitung 58 Sauerstoff aus einem Sauerstofftank 56 oder Luft, der bzw. die für die Versorgung des thermo-chemischen Reaktors 28 und der Brennkraftmaschine 12 erforderlich ist, und gasförmigen Brennstoff, gegebenenfalls unter Zwischenspeicherung eines Teiles des gasförmigen Brennstoffs als Methanol, über die zweite Reingasleitung 54 zur Versorgung der Brennkraftmaschine 12.

Mit der Erfindung werden somit mit hohem Wirkungsgrad aus kostengünstig zur Verfügung stehendem organischen Material Strom, Wärme und der Energieträger Methanol erzeugt. Die Anordnung der Vorrichtung in einem Kreislaufbetrieb erlaubt eine bedarfsgerechte Regelung der Stoff- und Energieströme. Auch wenn kein organisches Material zur Verfügung steht, kann die erfindungsgemäße Vorrichtung grundsätzlich jederzeit mit im Rahmen des Verfahrens erzeugtem Methanol oder anderen externen Brennstoffen betrieben werden, wodurch eine Energieversorgung sichergestellt wird. Das erfindungsgemäße Verfahren und die Vorrichtung sind daher insbesondere für einen Einsatz in Privathaushalten geeignet, wenn eine weitgehend autarke Energieversorgung erwünscht ist. Im Hinblick auf die Erzeugung von regenerativer Energie kann das erfindungsgemäße Verfahren auch im großen Maßstab als Alternative zu Blockheizkraftwerken eingesetzt werden.

## Patentansprüche

1.  Verfahren zur Erzeugung von Energie und Methanol mit den folgenden Schritten:

    1. Verbrennen von Brennstoff in einer Brennkraftmaschine (12) unter Erzeugung von mechanischer Energie und heißem kohlendioxid- und wasserdampfhaltigem Abgas,
    2. Reduktion der heißen Abgaskomponenten Kohlendioxid und Wasser in einem zugeführtes organisches Material enthaltenden thermo-chemischen Reaktor (28) zu einem Synthesegas, das Kohlenmonoxid und Wasserstoff enthält, wobei das organische Material oxidiert wird,
    3. Synthetisieren von Methanol aus dem Synthesegas und Abscheiden des Methanols
    4. Zuführen des in Schritt 3 nicht umgesetzten Synthesegases und/oder des synthetisierten Methanols in die Brennkraftmaschine (12).

2.  Verfahren nach Anspruch 1, bei dem synthetisiertes Methanol einem Methanoltank (50) oder einer Brennstoffzelleneinrichtung zugeführt wird.

3.  Verfahren nach Anspruch 1 oder 2, bei dem in Schritt 1 die mechanische Energie mittels eines Generators (16) in Strom umgewandelt wird.

**4.** Verfahren einem der Ansprüche 1 bis 3, bei dem das zugeführte organische Material zugeführte Biomasse, Kohle oder organische Abfälle ist.

**5.** Verfahren nach einem der Ansprüche 1 bis 4, bei dem zumindest ein Teil des in Schritt 2 erzeugten Wasserstoffs einer Brennstoffzelleneinrichtung zur Erzeugung von Strom zugeführt wird.

**6.** Verfahren nach einem der Ansprüche 1 bis 5, bei dem in einzelnen Verfahrensschritten entstehende Wärme einem Wärmespeicher (26) zugeführt wird.

**7.** Verfahren nach einem der Ansprüche 1 bis 6, bei dem ein Teil des in Schritt 1 erzeugten Abgases zur Erwärmung der organisches Material enthaltenden Umgebung verwendet wird.

**8.** Vorrichtung zur Erzeugung von Energie und Methanol, zur Durchführung des Verfahrens nach einem der Ansprüche 1 bis 7, mit einer Brennkraftmaschine (12), einem der Brennkraftmaschine nachgeordneten thermo-chemischen Reaktor (28) zur Reduktion von in der Brennkraftmaschine erzeugtem heißen kohlendioxid- und wasserdampfhaltigem Abgas mittels zugeführtem organischen Material zu einem Synthesegas, das Kohlenmonoxid und Wasserstoff enthält, und einem dem thermo-chemischen Reaktor (12) nachgeordneten Methanolreaktor (44) zur Synthese von Methanol aus dem Synthesegas des thermo-chemischen Reaktors, wobei eine Zuleitung von Syntheserestgas und/oder Methanol aus der Methanolsynthese in die Brennkraftmaschine (12) erfolgt.

**9.** Vorrichtung nach Anspruch 8, bei der ein Methanoltank (50) zur Speicherung des synthetisierten Methanols vorgesehen ist.

**10.** Vorrichtung nach Anspruch 8 oder 9, bei der eine Brennstoffzelleneinrichtung zur Erzeugung von Strom aus Methanol vorgesehen ist.

**11.** Vorrichtung nach einem der Ansprüche 8 bis 10, bei der ein durch die Brennkraftmaschine (12) antreibbarer Generator (16) zur Stromerzeugung vorgesehen ist.

**12.** Vorrichtung nach einem der Ansprüche 8 bis 11, bei der ein Wärmespeicher (26) zur Speicherung von mit der Vorrichtung erzeugter Wärme vorgesehen ist.

**Claims**

**1.** Process for generating energy and methanol comprising the following steps:

1. burning fuel in an internal combustion engine (12) thereby generating mechanical energy and hot exhaust gas containing carbon dioxide and water vapour,
2. reducing the hot exhaust gas components carbon dioxide and water in a thermochemical reactor (28) containing added organic material to produce a synthesis gas which contains carbon monoxide and hydrogen, the organic material being oxidised,
3. synthesising methanol from the synthesis gas and separating off the methanol
4. feeding the synthesis gas which did not react in step 3 and/or the synthesised methanol into the internal combustion engine (12).

**2.** Process according to claim 1, wherein synthesised methanol is fed into a methanol tank (50) or a fuel cell device.

**3.** Process according to claim 1 or 2, wherein in step 1 the mechanical energy is converted into electric current by means of a generator (16).

**4.** Process according to one of claims 1 to 3, wherein the added organic material is added biomass, coal or organic waste.

**5.** Process according to one of claims 1 to 4, wherein at least some of the hydrogen produced in step 2 is fed into a fuel cell device in order to generate electric current.

**6.** Process according to one of claims 1 to 5, wherein heat formed in individual steps of the process is fed into a heat store (26) .

**7.** Process according to one of claims 1 to 6, wherein some of the exhaust gas produced in step 1 is used to heat the environment containing the organic material.

**8.** Apparatus for generating energy and methanol, for carrying out the process according to one of claims 1 to 7, having an internal combustion engine (12), a thermochemical reactor (28) mounted downstream of the internal combustion engine for reducing hot exhaust gas containing carbon dioxide and water vapour produced in the internal combustion engine, by means of added organic material to form a synthesis gas which contains carbon monoxide and hydrogen, and a methanol reactor (44) downstream of the thermochemical reactor (12) for synthesising methanol from the synthesis gas from the thermochemical reactor, wherein residual synthesis gas and/or methanol from the synthesis of methanol is fed into the internal combustion engine (12).

**9.** Apparatus according to claim 8, wherein a methanol tank (50) is provided for storing the synthesised methanol.

**10.** Apparatus according to claim 8 or 9, wherein a fuel cell device is provided for generating electric current from methanol.

**11.** Apparatus according to one of claims 8 to 10, wherein a generator (16) which can be driven by the internal combustion engine (12) is provided for generating electric current.

**12.** Apparatus according to one of claims 8 to 11, wherein a heat store (26) is provided for storing heat generated by the apparatus.


**Revendications**

**1.** Procédé pour produire de l'énergie et du méthanol, par les étapes suivantes :

> 1. combustion d'un combustible dans un moteur à combustion (12) en produisant de l'énergie mécanique et des gaz d'échappement chaud contenant du gaz carbonique et de la vapeur d'eau,
> 2. réduction des composants chauds des gaz d'échappement, à savoir gaz carbonique et vapeur d'eau, dans un réacteur (28) thermochimique contenant du matériau organique amené, pour former un gaz de synthèse qui contient du monoxyde de carbone et de l'hydrogène, le matériau organique étant oxydé,
> 3. synthétisation de méthanol à partir du gaz de synthèse et précipitation du méthanol,
> 4. amenée à un moteur à combustion (12) du gaz de synthèse non converti au cours de l'étape 3 et/ou du méthanol synthétisé.

**2.** Procédé selon la revendication 1, dans lequel le méthanol synthétisé est amené à un réservoir de méthanol (50) ou à un système de cellules à combustible.

**3.** Procédé selon l'une ou l'autre des revendications 1 et 2, dans lequel, au cours de l'étape 1, l'énergie mécanique est convertie en courant au moyen d'un générateur (16).

**4.** Procédé selon l'une des revendications 1 à 3, dans lequel le matériau organique amené est de la biomasse, du charbon ou des déchets organiques amené(s).

**5.** Procédé selon l'une des revendications 1 à 4, dans lequel au moins une partie de l'hydrogène produit au cours de l'étape 2 est amenée à un système de cellules à combustible pour produire du courant.

**6.** Procédé selon l'une des revendications 1 à 5, dans lequel de la chaleur produite au cours d'étapes de procédé individuelles est amenée à un accumulateur de chaleur (26).

**7.** Procédé selon l'une des revendications 1 à 6, dans lequel une partie des gaz d'échappement produits au cours de l'étape 1 est utilisée pour chauffer l'environnement contenant le matériau organique.

**8.** Dispositif de production d'énergie et de méthanol, pour mettre en oeuvre le procédé selon l'une des revendications 1 à 7, comportant un moteur à combustion (12), un réacteur (28) thermochimique associé en aval au moteur de combustion pour réduire, au moyen de matériau organique amené, des gaz d'échappement produits dans le moteur à combustion et contenant du gaz carbonique et de la vapeur d'eau, pour former un gaz de synthèse qui contient du monoxyde de carbone et de l'hydrogène, et un réacteur de méthanol (44) associé en aval au réacteur thermochimique (12), pour effectuer une synthèse de méthanol à partir du gaz de synthèse du réacteur thermochimique, une amenée de gaz résiduel de synthèse et/ou de méthanol ayant lieu à partir de la synthèse de méthanol vers le moteur à combustion (12) .

**9.** Dispositif selon la revendication 8, dans lequel il est prévu un réservoir de méthanol (50) pour accumuler le méthanol synthétisé.

**10.** Dispositif selon l'une ou l'autre des revendications 8 et 9, dans lequel un système de cellules à combustible est prévu pour produire du courant à partir du méthanol.

**11.** Dispositif selon l'une des revendications 8 à 10, dans lequel il est prévu un générateur (16) qui peut être entraîné par le moteur à combustion (12) pour produire du courant.

**12.** Dispositif selon l'une des revendications 8 à 11, dans lequel un accumulateur de chaleur (26) est prévu pour accumuler la chaleur produite avec le dispositif.